# EUROPEAN PATENT APPLICATION

(11) **EP 4 692 341 A1**
(43) Date of publication of application: **11.02.2026**
(21) Application number: 24787842.4
(22) Date of filing: 06.03.2024
(51) Int. Cl.: C12N 9/64

(54) **EXPRESSION AND PURIFICATION METHOD FOR BATROXOBIN AND USE OF SAME**

(30) Priority: 14.04.2023 CN 202310399963
(71) Applicant: Shanghai Tenry Pharmaceutical Co., Ltd., Fengxian District, Shanghai 201400 (CN)
(72) Inventor: ZHANG, Yan, Shanghai 201400 (CN); LI, Qing, Shanghai 201400 (CN); TANG, Fang, Shanghai 201400 (CN); GUO, Senlin, Shanghai 201400 (CN); GUAN, Lufan, Shanghai 201400 (CN); LI, Zhengwu, Shanghai 201400 (CN)
(74) Representative: ABG Intellectual Property Law, S.L.
(86) International application number: PCT/CN2024/080242
(87) International publication number: WO 2024/212736

(57) **Abstract**

Provided is an expression and purification method for recombinant batroxobin. By means of adjustment and optimization on the pH value and conductivity of a culture medium supernatant, a target protein can well bind to a chromatography filler, whereas most of impurity proteins cannot bind to the filler and thus flow through same, thus improving the purity of a collected liquid; therefore, the method can suppress the activity of a hydrolase in a fermentation supernatant, and further ensures no flow-through of the target protein, thereby ensuring a high yield. Further, integrating cation chromatography, anion chromatography, unidirectional tangential-flow filtration and concentration and gel filtration chromatography for purification obtains high-purity active batroxobin protein. A simple salt-gradient elution mode is replaced by a salt-gradient and pH-gradient composite elution mode, thus greatly improving the protein purity. The purification method uses a continuous-flow purification process, and an intermediate product does not need to undergo pH value and conductivity regulation, dilution, liquid change and other operations, thereby achieving advantages of shortened process time, improved enzyme activity, reduced storage tanks, workers and plant area, etc. In-vitro and in-vivo verification shows that the batroxobin purified by the method of the present invention has blood coagulation effect similar to that of commercially available natural batroxobin, thereby achieving extensive application prospects.

## Description

### Technical Field

The invention relates to the field of biological engineering, and particularly to an expression and purification method for batroxobin and use of same.

### Background Art

Batroxobin is a serine proteolytic enzyme (thrombin like enzyme) firstly isolated from the venom of Bothrops atrox by Austrian scholar Von Klobusitzky in 1963. Although batroxobin protein has only one peptide chain, there are 12 cysteine residues in the batroxobin molecule, forming six intramolecular disulfide bonds; there is also glycosylation modification, which has two N-glycosylation sites within its molecule: ASnl46-ASn"17-Thr'" and Asn225-Lys226-Thr227. Although researchers have gained a relatively good understanding of the properties of batroxobin, molecular biology research on this snake venom component has been progressing slowly.

Batroxobin is a serine protease-like enzyme, and its specific substrate is fibrinogen. Unlike thrombin, Batroxobin only cleaves the A chain of fibrinogen and does not act on the B chain. When it hydrolyzes the Arg16-Gly17 peptide bond in the A chain of the plasma fibrinogen, it can release fibrinogen peptide A, which can rapidly convert fibrinogens in the blood into fibrins, and then these fibrins can be aggregated into loose thrombi that are easily to be hydrolyzed by fibrinolytic enzymes to close the wound and achieve the effect of rapid hemostasis. At the same time, it does not activate blood coagulation factor XIII in vivo, and the side chains of fibrin clots produced by its hydrolysis cannot be crosslinked and are easily degraded by fibrinolytic enzymes, so it will not cause thrombosis in the blood system.

This soft clot is easily degraded by hydrolytic enzymes, leading to a decrease in the concentration of fibrinogen in the blood, thereby improving blood viscosity and hydrodynamic properties of the blood, playing the role of a defibrase. Based on these biochemical characteristics, batroxobin has been successfully developed into hemostatic and fibrin-lowering drugs. Batroxobin is replacing human thrombin as a hemostatic drug in Europe. The production cost of extracting batroxobin from snake venom is too high. The single source, low yield, and high price have always been factors restricting the widespread application of batroxobin.

The production of proteins rich in disulfide bonds and glycosylation modifications by means of genetic engineering has always been a technical challenge, especially the production of serine hydrolase-like molecules with multiple pairs of disulfide bonds. This is because the pairing error rate of disulfide bonds is very high, and the amount of snake venom thrombin expressed in E. coli is very small, almost all of which are inclusion bodies.

A high rate of correct disulfide bond pairing can be ensured in eukaryotic expression systems (yeast, CHO, insect cells, etc.). Eukaryotic expression systems can ensure the correct rate of disulfide bond pairing, and protein folding and assembly occur on the endoplasmic reticulum, with the synergistic effect of molecular chaperones, proteins with good biological activity can be produced. Yeast has the characteristics of prokaryotic organisms such as fast cell growth of prokaryotic expression system, easy cultivation, and simple genetic manipulation; it also has the functions of correct processing, modification, and reasonable spatial folding of expressed proteins in eukaryotes, and its properties are more stable than those of prokaryotic expressed proteins, which is very conducive to the expression of eukaryotic genes. It can effectively overcome the shortcomings of the E. coli system, such as the lack of protein post-translational processing and modification and the difficulty of protein renaturation in the later stage. Therefore, yeast expression systems are receiving more and more attention and utilization. In 2004, You Weon-Kyoo from South Korea reported the expression of batroxobin in Pichia pastoris, with an expression level of 3.431 NIH/mL, and 7mg of batroxobin can be purified from each liter of fermentation broth.

Due to the structural characteristics of batroxobin itself, the activity of batroxobin tends to decrease during the production process using genetic engineering fermentation. The main reason is that proteases in the yeast host system may degrade the accumulated batroxobin in the fermentation broth, thereby affecting the yield and enzyme activity of batroxobin.

Therefore, there are deficiencies in the prior art such as low activity of batroxobin obtained by fermentation and insufficient enzyme degradation.

### Summary of the Invention

The purpose of the present invention is to provide an expression and purification method for batroxobin with respect to the low yield and low enzyme activity of purified batroxobin in the prior art.

To solve the above technical problems, the present invention/utility model adopts the following technical solutions:
The present invention discloses an expression and purification method for batroxobin, comprising the following steps
1) Taking the supernatant of the yeast fermentation broth of batroxobin, and adjusting pH value and conductivity;
2) Performing chromatography over a cation resin;
3) Performing chromatography over an anion resin;
4) Performing unidirectional tangential-flow filtration and concentration;
5) Performing gel filtration chromatography to concentrated protein solution; and
6) Eluting and collecting target protein peak to obtain purified batroxobin.

Preferably, in step 1), the pH value is adjusted to 5.8, and the conductivity is adjusted to 6mS/cm.

Preferably, the elution operation of chromatography in step 2) is to first remove impurity proteins with low salt buffer, to elute and remove pigments by using buffer PB with a pH adjusted to 8.0, and finally to elute the target protein by using buffer Tris-HCl with a pH of 9.0.

Preferably, the low salt buffer is a 0.15M NaCl buffer adjusted to pH 6.0 with NaAc/HAc.

Preferably, the elution operation of chromatography in step 3) uses an elution buffer of 50mM Tris-HCl+0.5M NaCl, pH 9.0.

Preferably, the elution operation of chromatography in step 5) uses an elution buffer of 20mM PB+0.15M NaCl, pH 6.0.

Preferably, the cation resin used for chromatography in step 2) is SP Sepharose FF.

Preferably, the anion resin used for chromatography in step 3) is Q Sepharose FF.

Preferably, the filler in the gel filtration used for chromatography in step 5) is Superdex 75 PG.

Preferably, the method further comprises an operation of determining the protein concentration of the batroxobin protein obtained via purification.

During the purification process of batroxobin of the present invention, by means of adjustment and optimization on the pH value and conductivity of a culture medium supernatant, a target protein can well bind to a chromatography filler, whereas most of impurity proteins cannot bind to the filler and thus flow through same, thus improving the purity of a collected liquid; therefore, the method can suppress the activity of a hydrolase in a fermentation supernatant, and further ensures no flow-through of the target protein, thereby ensuring a high yield. A simple salt-gradient elution mode is replaced by a salt-gradient and pH-gradient composite elution mode, thus greatly improving the protein purity. Further, integrating cation chromatography, anion chromatography, unidirectional tangential-flow filtration and concentration and gel filtration chromatography for purification obtains high-purity active batroxobin protein. The purification method of the present invention uses a continuous-flow purification process, and an intermediate product does not need to undergo pH value and conductivity regulation, dilution, liquid change and other operations, thereby achieving advantages of shortened process time, improved enzyme activity, reduced storage tanks, workers and plant area, etc. In-vitro and in-vivo verification shows that the batroxobin purified by the method of the present invention has blood coagulation effect similar to that of commercially available natural batroxobin, thereby achieving extensive application prospects.

### Brief Description of the Drawings

Fig. 1. PCR screening and identification image of transformed yeast colonies.
Fig. 2. Plot of coagulation effect of batroxobin purified from culture medium under different pH conditions.
Fig. 3. Plot of coagulation effect of batroxobin purified from culture media with different conductivities.
Fig. 4. Plot of coagulation effect of adding anion resin and unidirectional tangential-flow filtration on purified batroxobin.
Fig. 5. SDS electrophoresis detection image of purified batroxobin protein.
Fig. 6. Relative molecular weight analysis spectrum of the intact protein.
Fig. 7. Relative molecular weight analysis spectrum after reducing de-N-glycosylation.
Fig. 8. Plot of the coagulation effect of purified recombinant batroxobin on mouse bleeding.

### Detailed Description of Examples

The Detailed Description of Examples of the present invention will be described in detail below in combination with the Drawings. It should be understood that the Detailed Description of Examples described herein are only for illustrating and explaining the present invention, and are not intended to limit the present invention.

### Example 1. Construction and transformation of yeast expression vector containing batroxobin gene

### 1) Cloning of the gene encoding batroxobin

Using the nucleic acid sequence of Batroxobin (J02684.1) disclosed in GeneBank, the gene sequence was first artificially synthesized. Based on the information of the gene, the information about the enzyme cleavage sites in the gene, and the like, primer sequences for amplifying the gene encoding batroxobin were designed, and XhoI and SacII enzyme cleavage site sequences were added to both ends of the primers. The designed amplification primer sequences were as follows:
Sense primer: 5'- aactcgaggtcattgga ggtgatgaat -3';
Anti-sense primer: 5'- aaccgcggcgggcaagt cgcagttt -3'.

PCR amplification was performed on the synthesized batroxobin gene J02684.1 using the aforementioned amplification primers.

The PCR amplification reaction conditions were as follows:
95 °C for 3 min; 95 °C for 40s, 53 °C for 30s, 72 °C for 1 min, 35 cycles; and 72 °C for 15 min.

The obtained amplification product was stored at 4 °C for future use.

### 2) Construction and Transformation of Yeast Expression Vector

The PCR amplified product from step 1) was purified and recovered, and then double digested with XhoI and SacII, and at same time the yeast expression vector pPICZαA was also double digested with XhoI and SacII. After the digested products were recovered separately and then ligated with T4 ligase, and the ligated products were transformed into E. coli BL21. The recombinant bacteria were identified, the recombinant plasmid was extracted, and the positive clone was screened by double digestion and PCR amplification of the target gene, and the recombinant plasmid was named as pPICZ α A-Batr.

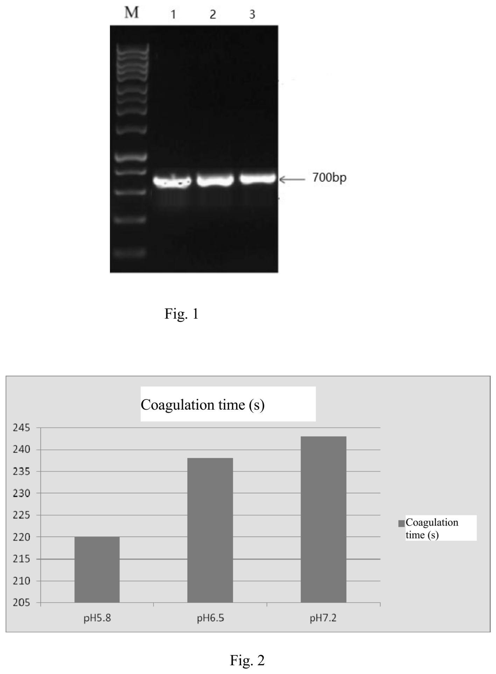

The pPICZαA-Batr plasmid was extracted, linearized with Sac I, and then transformed into Pichia pastoris X-33 by electrotransformation. All transformants were coated on YPD+Zeocin (100 µ g/ml) medium and incubated at 30 °C for 4-5 days, the fast-growing and plump colonies were selected for colony PCR identification, and the above amplification primers were used for the PCR identification. Among them, the identification diagram of yeast colonies by PCR was shown in Fig. 1. The identified clones of the positive colony were stored for future use.

### Example 2. Fermentative expression of engineered yeast X-33/pPICZαA-Batr

### 1) Preliminary screening of enzyme expression levels in positive colonies

Positive clones were selected, first inoculated in glycerol composite medium (BMGY) and shaken overnight, then centrifuged to remove the culture medium. The precipitated bacteria were re-suspended in BMMY and diluted to OD₆₀₀ of 1 for induction of expression. Samples were taken every 12 hours and methanol was added to the final concentration of 1%, and the supernatant was collected by centrifugation after 72 h. Each sample was centrifuged and the supernatant was retained for preliminary determination of thrombin activity, the results of which were shown in Table 1. The clones of colonies with high thrombin activity were preliminarily screened and obtained for fermentation in a fermentation tank. The method for determining thrombin activity was as follows:
0.2ml of human-citrate coagulation plasma was taken and added to a 96-well plate, keeping at 37 °C for 3 minutes. 0.2ml of sample solution preheated at 37 °C was added, immediately shaken to mix evenly, and timed, and the plasma coagulation condition was begun to check at 40s. The initial coagulation time was recorded, and 3 tubes were determined simultaneously. The error should be less than 20s. If the initial coagulation time was less than 40s, the concentration of the test sample solution that coagulated within (60 ± 20) seconds was recorded, when the test sample solution was diluted several times. Under the above conditions, the amount of enzyme that could coagulate 0.2ml of human-citrate anticoagulant plasma in 60s was defined as one enzyme activity unit.

**Table 1: Preliminary thrombin activity determination of the expression induced by different positive clone of engineered yeast X-33/pPICZαA-Batr for 36 hours**

| Colony Number | 1 | 2 | 3 | 4 | 5 | 6 |
|---|---|---|---|---|---|---|
| Coagulation time (S) | 262 | 231 | 247 | 289 | 255 | 269 |

It could be seen from the determination results that the activity of batroxobin expressed in colony 2 was the highest, and the coagulation time was the shortest. Colony 2 was used for later fermentation and purification operations.

### 2) Fermentation of yeast X-33/pPICZαA-Batr-2

The stored colony 2 was inoculated into a test tube containing 5mL of BMGY yeast culture medium and incubated overnight. The culture medium in the test tube was transferred into a shake flask containing 180mL BMGY culture medium for proliferation as seed solution, and then transferred into a biological fermentation tank containing 5L BMMY fermentation broth. The pilot-scale fermentation was conducted using the conventional method of methanol yeast fermentation and the supernatant was collected for purification and future use.

### Example 3. Purification of yeast X-33/pPICZαA-Batr-2 fermentation broth

### 3-1) Preliminary purification of the fermentation supernatant: adjusting different pH values of the fermentation broth

Following the conventional purification method for yeast fermentation broth proteins, the different pH values of the fermentation broth were first adjusted to observe the activity of the purified enzyme.
①200mL of supernatant from the fermentation broth were taken, respectively, and the pH values were adjusted to 5.8, 6.5, and 7.2, respectively;
②Chromatography was performed on the above supernatants over a cation filler, which was SP Sepharose FF. First, the impurity proteins were removed with low salt (NaAc/HAc, 0.15M NaCl, pH 6.0), the buffer (PB) with pH adjusted to 8.0 was used to elute and remove pigments, and finally the buffer (Tris-HCl) with pH of 9.0 was used to elute the target protein;
③ The protein eluent was concentrated using an ultrafiltration tube (10Kd) for later use;
④ The concentrated protein solution was subjected to gel filtration chromatography, and the gel filtration filler used was Superdex 75 PG. The chromatography column was first washed with an equilibration buffer (20mM PB+0.15M NaCl, pH6.0). After equilibration, the unidirectional tangential-flow filtration collection solution was loaded, with loading volume ≤ 5% of the column volume, and the target protein peak was collected.
⑤ The concentration of the protein in the eluent was determined and was adjusted to the consistent concentration. The method in Example 2 was used to determine the enzyme activity of the collected batroxobin. The determination results were shown in Fig. 2.

It could be seen from the test results that increasing the pH of the loading sample to around 5.8 allowed the target protein to bind well to the chromatography filler, while most impurity proteins cannot bind to the filler and flowed through same, thus improving the purity and yield of the collection solution and thus enhancing enzyme activity.

### 3-2) Preliminary purification of the fermentation supernatant: adjusting the conductivity of the fermentation broth

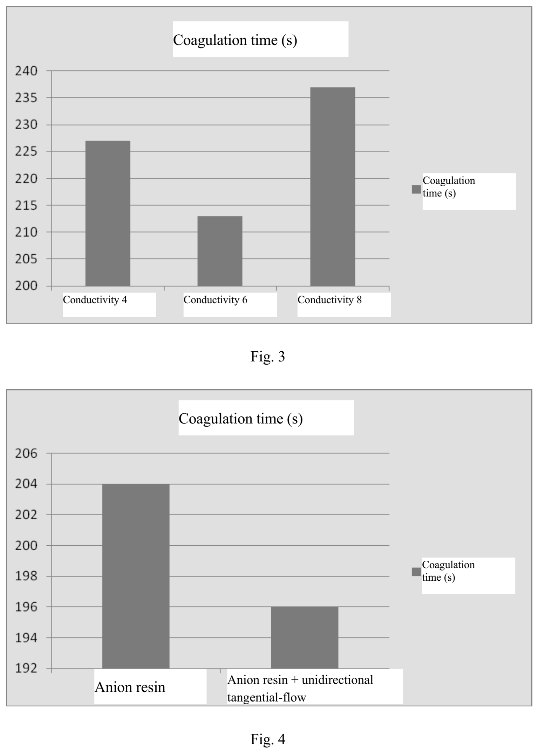

The pH value of the fermentation broth was adjusted to around 5.8, further the conductivity was adjusted respectively to observe the activity of the purified enzyme.
①200mL of supernatants from the fermentation broth were taken, respectively, the pH values were adjusted to 5.8, respectively, and the conductivities of the culture media were adjusted to 4mS/cm, 6mS/cm, and 8mS/cm, respectively;
② Chromatography was performed on the above supernatant over a cation filler, which was SP Sepharose FF. First, impurity proteins were removed with low salt (NaAc/HAc, 0.15M NaCl, pH 6.0), the buffer (PB) with pH adjusted to 8.0 was used to elute and remove pigments, and finally the buffer (Tris-HCl) with pH of 9.0 was used to elute the target protein;
③ The protein eluent was concentrated using an ultrafiltration tube (10Kd) for later use;
④ The concentrated protein solution was subjected to gel filtration chromatography, and the gel filtration filler used was Superdex 75 PG. The chromatography column was first washed with an equilibration buffer (20mM PB+0.15M NaCl, pH6.0). After equilibration, the concentrated solution was loaded, with loading volume ≤ 5% of the column volume, and the target protein peak was collected.
⑤ The concentration of the protein in the eluent was determined and was adjusted to the consistent concentration. The method in Example 2 was used to determine the enzyme activity of the collected batroxobin. The determination results were shown in Fig. 3.

It could be seen from the test results that the conductivity of the loading sample adjusted to around 6 can not only suppress the activity of a hydrolase in the fermentation supernatant, but also ensure no flow-through of the target protein, ensuring a high yield and thus improving activity of the enzyme.

### 3-3) Preliminary purification of the fermentation supernatant: adding anion exchange chromatography step for purification

Anions easily bound to pigments in the culture medium, and further the salt gradient linear elution mode was utilized to increase the purity of the target protein to around 95%.
①200mL of supernatants from the fermentation broth were taken, respectively, the pH values were adjusted to 5.8, respectively, and the conductivities of the culture media were adjusted to 6mS/cm;
② Chromatography was performed on the above supernatant over a cation filler, which was SP Sepharose FF. First, impurity proteins were removed with low salt (NaAc/HAc, 0.15M NaCl, pH 6.0), the buffer (PB) with pH adjusted to 8.0 was used to elute and remove pigments, and finally the buffer (Tris-HCl) with pH of 9.0 was used to elute the target protein;
③ The cation eluent was loaded onto an anion resin, which was Q Sepharose FF. After completion, the chromatography column is washed with the equilibration buffer, and then eluted with an elution buffer (50mM Tris-HCl+0.5M NaCl, pH 9.0) for 0-100% elution of 15CV. The elution peak of the target protein was collected.

After the chromatography, the chromatography filler was cleaned and disinfected: after elution, the anion chromatography filler still carried obvious pigments, which cannot be completely removed with 0.5M NaOH and 1M NaCl. After multiple uses, the pigments will be accumulated, leading to darker color of the collection solution, decreased filler yield, poor column efficiency, and difficulty in packing, and the like. Thus, first, 0.5M H₃PO₄ was used to elute the residual pigments on the filler, with a retention time of 8-10 minutes. Then, 0.5M NaOH+1M NaCl was used to regenerate, clean and disinfect the filler, so that the filler returned to its initial milky white color.

### Process description:

The pH of the centrifuged supernatant was adjusted to 5.8-6.0 and the conductivity to 5-6 mS/cm using the injection water and 10% glacial acetic acid.

The cation chromatography column was first washed with an equilibration buffer (20mM NaAc/HAc, pH 6.0), and then the sample with adjusted pH and conductivity was loaded. After the sample loading, the chromatography column was washed with equilibrium buffer and the elution process began: first, a rinsing buffer 1 (20mM NaAc/HAc+0.15M NaCl, pH 6.0) was used to elute impurity proteins, then an rinsing buffer 2 (20mM PB, pH 8.0) was used to elute pigments and other impurities, and finally an elution buffer (50mM Tris-HCl, pH 9.0) was used for elution, and the elution peak was collected.

The anion chromatography column was first washed with an equilibration buffer (50mM Tris HCl, pH 9.0), and the cation exchange chromatography collection solution was loaded. After the sample loading, the chromatography column was washed with equilibration buffer, and then an elution buffer (50mM Tris HCl+0.5M NaCl, pH 9.0) was used for 0-100% elution of 15CV. The elution peak of the target protein was collected.

The chromatography filler was first equilibrated to pH 6.0 with an equilibrium buffer solution (20mM NaAc/HAc, pH 6.0). Then, the chromatography column was cleaned with 0.5M H₃PO₄, with a retention time of 8-10 minutes. After equilibration with the above equilibrium buffer solution, the filler was regenerated and disinfected with 0.5M NaOH+1M NaCl, with a retention time of 30 minutes.
④ The protein eluent was concentrated using an ultrafiltration tube (10Kd) for later use;
⑤ The concentrated protein solution was subjected to gel filtration chromatography, and the gel filtration filler used was Superdex 75 PG. The chromatography column was first washed with an equilibration buffer (20mM PB+0.15M NaCl, pH6.0). After equilibration, the concentrated solution was loaded, with loading volume ≤ 5% of the column volume, and the target protein peak was collected.
⑥ The concentration of the protein in the eluent was determined and was adjusted to the consistent concentration. The method in Example 2 was used to determine the enzyme activity of the collected batroxobin. The determination results were shown in Fig. 4.

### 3-4) Preliminary purification of the fermentation supernatant: adding an unidirectional tangential-flow filtration step

The unidirectional tangential-flow filtration was easy to operate and suitable for amplification, can quickly concentrate the target protein, can control the loading volume as required for subsequent operations, and reduce the number of cycles and filler volume of gel filtration chromatography.
① 200mL of supernatants from the fermentation broth were taken, respectively, the pH values were adjusted to 5.8, respectively, and the conductivities of the culture media were adjusted were adjusted to 6mS/cm;
② Chromatography was performed on the above supernatant over a cation filler, which was SP Sepharose FF. First, impurity proteins were removed with low salt (NaAc/HAc, 0.15M NaCl, pH 6.0), the buffer (PB) with pH adjusted to 8.0 was used to elute and remove pigments, and finally the buffer (Tris-HCl) with pH of 9.0 was used to elute the target protein;
③ The cation eluent was loaded onto an anion resin, which was Q Sepharose FF. After completion, the chromatography column is washed with the equilibration buffer, and then eluted with an elution buffer (50mM Tris-HCl+0.5M NaCl, pH 9.0) for 0-100% elution of 15CV. The elution peak of the target protein was collected.;
   The anion chromatography filler was first equilibrated to pH 6.0 with an equilibrium buffer solution (20mM NaAc/HAc, pH 6.0). Then, the chromatography column was cleaned with 0.5M H₃PO₄, with a retention time of 8-10 minutes. After equilibration with the above equilibrium buffer solution, the filler was regenerated and disinfected with 0.5M NaOH+1M NaCl, with a retention time of 30 minutes.
④ The unidirectional tangential-flow filtration, a membrane package (0.11m2, 10Kd) was first washed with the equilibration buffer (50mM Tris-HCl, pH 9.0), and each stage of the membrane packages was connected in series, and the same stage membrane package was connected in parallel. After equilibration, the anion exchange chromatography collection solution was loaded, and the permeate end was the waste liquid. The final reflux end sample was collected, and the membrane package was washed with the injection water and 0.5M NaOH.
⑤ The concentrated protein solution was subjected to gel filtration chromatography, and the gel filtration filler used was Superdex 75 PG. The chromatography column was first washed with equilibration buffer (20mM PB+0.15M NaCl, pH6.0). After equilibration, unidirectional tangential-flow filtration collection solution was loaded, with loading volume ≤ 5% of the column volume, and the target protein peak was collected.
⑥ The concentration of the protein in the eluent was determined and was adjusted to the consistent concentration. The method in Example 2 was used to determine the enzyme activity of the collected batroxobin. The determination results were shown in Fig. 4.

It can be seen from the detection results in Fig. 4 that further increasing the adsorption and elution of anion resin and unidirectional tangential-flow filtration during the purification process could further increase the activity of the purified batroxobin. The reason for this might be that the purification step reduced the contents of impurity proteins, further increasing the concentration of batroxobin, and to some extent reducing the interference of impurity proteins on batroxobin.

### Example 4. SDS electrophoresis detection of purified batroxobin protein

Electrophoresis detection was performed on the batroxobin purified and prepared by Method 3-4 in Example 3, with the following main operations.

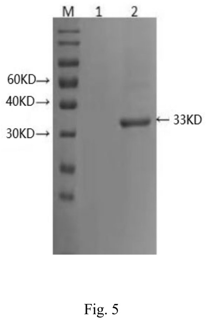

The purified sample was taken and electrophoresis detection was conducted in 15% concentration of polypropylene amide gel. The protein sample was mixed with the loading buffer in a 4:1 ratio and boiled in a bath for 5 minutes to denature the protein. After cooling at room temperature, a microporous loading device was used to load the sample. The electrophoresis voltage was 80-100V for concentrated gel and 100-120V for separation gel. The electrophoresis detection results were shown in Fig. 5. Among them, band 1 is the negative control, and band 2 is the batroxobin prepared and purified by Method 3-4 in Example 3. It can be seen that the protein prepared by this purification method had no other impurity proteins and a high purity.

### Example 5. Relative molecular weight detection of purified batroxobin protein

The theoretically calculated molecular weight of batroxobin is 25.6KD, but the molecular weight of the batroxobin protein secreted and expressed by the methanol yeast engineering bacteria constructed by the inventors was determined to be about 33KD by reduction electrophoresis, which was inconsistent with the theoretically calculated value. The reason for this situation was that there are two N-glycosylation sites in the batroxobin molecule. According to the general rules of research on most glycosylated proteins, each glycosylation can increase the molecular weight of the protein by about 0.3~0.5KD. Therefore, this difference in molecular weight should be the result from glycosylation modification, as shown in the detection results of mass spectrometry below for details.

### 1)De-N-glycosylation protein treatment

40ug of purified samples each was taken and DTT was added, which was reduced at 55 °C for 60 minutes and cooled to room temperature. 1 uL of PNGase F was added, mixed well, and incubated overnight at 37 °C.

### 2) Intact protein treatment

6M guanidine hydrochloride was added into 40ug purified sample for denaturation treatment. DTT was added to a final concentration of 50mM, which underwent reduction denaturation at 100 °C for 10 minutes, was diluted and subjected to liquidquality detection.

### 3) The parameters of high performance liquid chromatography

Separation was performed using a UPLC (Acquity UPLC I-Class, Waters) liquid phase system. The mobile phase A was a 0.1% FA aqueous solution, and B was a 0.1% FA acetonitrile solution. 1 µL of sample was loaded by an automatic sampler, and then separated by a chromatography column (BioResolve RP Column, 450 Å, 2.7 µ m, 2.1X50mm, Waters) with a flow rate of 0.3ml/min, UV detection wavelength of 280nm, column temperature of 80 °C, and analysis time of 10 minutes. The separation gradients were as follows: from 0 minutes to 1 minute, the linear gradient of liquid B was maintained at 15%, and from 1 minute to 7 minutes, the linear gradient of liquid B ranged from 15% to 60%; from 7 minutes to 7.5 minutes, the linear gradient of liquid B ranged from 60% to 90%; from 7.5 minutes to 8 minutes, the liquid B was maintained at 90%; from 8 minutes to 10 minutes, the liquid B was maintained at 15%.

### 4) Mass spectrometry parameters

A XevoG2-XS Q-Tof mass spectrometer (Waters) was used for mass spectrometry analysis. Analysis duration: 10 minutes, detection method: positive ion, parent ion scanning range: 500-4000m/z.

### 5) Data analysis

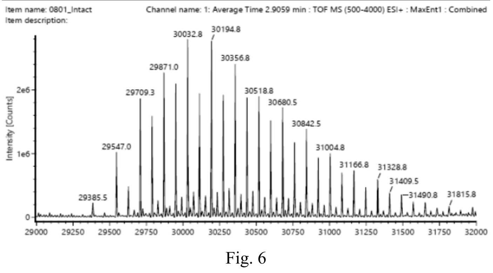

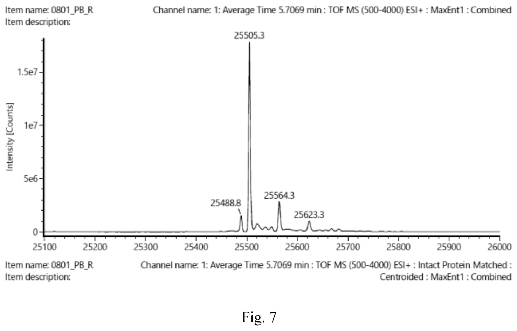

The raw data was processed by UNIFI (1.8.2, Waters) software. The intact protein relative molecular weight analysis spectrum was shown in Fig. 6, and the relative molecular weight analysis spectrum after reducing de-N-glycosylation was shown in Fig. 7.

The relative molecular weight of the purified sample's intact protein was 30,195Da. Among them, N-glycosylation modification mainly involved high mannose (M10-M15, etc.) with different data, and some mannose underwent phosphorylation modification (Phosphate); and the relative molecular weight after reducing de-N-glycosylation was 25,505 Da.

### Example 6. Determination of enzyme activities between purified recombinant batroxobin and commercially available natural batroxobin

Commercially available batroxobin was taken and dissolved in buffer (20mM PB+0.15M NaCl, pH 6.0), and the protein concentration determined with conventional methods was consistent with the purified recombinant batroxobin prepared by Method 3-4 in Example 3.

The method for determining enzyme activity refers to the method in Example 2: the method for determining thrombin activity was as follows:
0.2ml of human-citrate coagulation quality control plasma was taken and added to a 96-well plate, keeping at 37 °C for 3 minutes. 0.2ml of sample solution preheated at 37 °C was added, immediately shaken to mix evenly, and timed, and the plasma coagulation condition was begun to check at 40s. The initial coagulation time was recorded, and 3 tubes were determined simultaneously. The error should be less than 20s. If the initial coagulation time was less than 40s, the concentration of the test sample solution that coagulated within (60 ± 20) seconds was recorded, when the test sample solution was diluted several times. Under the above conditions, the amount of enzyme that could coagulate 0.2ml of human-citrate anticoagulant plasma in 60s was defined as one enzyme activity unit. The determination results were shown in Table 2.

**Table 2: Enzyme activity determination of recombinant batroxobin**

| Enzyme type | Recombinant batroxobin | Commercially available batroxobin |
|---|---|---|
| Coagulation time (S) | 193 | 176 |

### Example 7. The effect of purified recombinant batroxobin and commercially available batroxobin on mouse bleeding

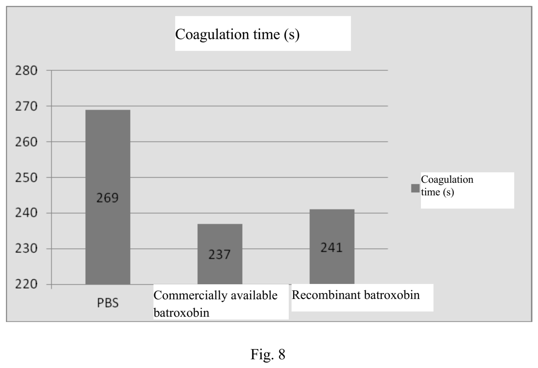

The identification of in vivo activity of recombinantly prepared batroxobin was determined by measuring the coagulation time of mice after tail amputation. The specific operations were as follows:
1) Nine healthy mice were taken and randomly divided into three groups, with three mice in each group;
2) The corresponding buffer solutions were intravenously into mice, with one group injected with PB buffer as a negative control, one group injected with commercially available batroxobin (dissolved in buffer solution (20mM PB+0.15M NaCl, pH 6.0)), and the last group injected with purified recombinant batroxobin prepared by Method 3-4 in Example 3;
3) Half an hour after injection, the tail was broken with scissors at a distance of 0.5cm from the tail tip of the mouse, and the timing was done immediately after the blood flowed out on its own. The average coagulation time of each group of mice was calculated. The determination results were shown in Fig. 8.

It could be seen from the determination results that the recombinantly purified batroxobin prepared by the method of the present invention has achieved or is close to the coagulation effect of natural batroxobin, and also has a certain coagulation effect in vivo.

The present invention illustrates the process method of the present invention via the above examples, but the present invention is not limited to the above process steps, that is, it does not mean that the present invention must rely on the above process steps to be implemented. Those skilled in the art should understand that any improvement to the present invention, equivalent substitution of raw materials used in the present invention, addition of auxiliary ingredients, selection of specific manners, etc., are all fallen into the scope of protection and disclosure of the present invention.

## Claims

1. An expression and purification method for recombinant batroxobin, **characterized by**, comprising the following steps:
1) Taking the supernatant of the yeast fermentation broth of recombinant batroxobin, and adjusting pH value and conductivity;
2) Performing chromatography over a cation resin;
3) Performing chromatography over an anion resin;
4) Performing unidirectional tangential-flow filtration and concentration;
5) Performing gel filtration chromatography to concentrated protein solution; and
6) Eluting and collecting target protein peaks to obtain purified batroxobin.

2. The purification method according to claim 1, **characterized in that** in step 1), the pH value is adjusted to 5.8 and the conductivity is adjusted to 6mS/cm.

3. The purification method according to claim 1, **characterized in that** the elution operation of chromatography in step 2) is to first remove impurity proteins with low salt buffer, to elute and remove pigments by using buffer PB with a pH adjusted to 8.0, and finally to elute the target protein by using buffer Tris-HCl with a pH of 9.0.

4. The purification method according to claim 3, **characterized in that** the low salt buffer is a 0.15M NaCl buffer adjusted to pH 6.0 with NaAc/HAc.

5. The purification method according to claim 1, **characterized in that** the elution operation of chromatography in step 3) uses an elution buffer of 50mM Tris-HCl+0.5M NaCl, pH 9.0.

6. The purification method according to claim 1, **characterized in that** the elution operation of chromatography in step 5) uses an elution buffer of 20mM PB+0.15M NaCl, pH 6.0.

7. The purification method according to claim 1, **characterized in that** the cation resin used for chromatography in step 2) is SP Sepharose FF.

8. The purification method according to claim 1, **characterized in that** the anion resin used for chromatography in step 3) is Q Sepharose FF.

9. The purification method according to claim 1, **characterized in that** the filler in the gel filtration used for chromatography in step 5) is Superdex 75 PG.

10. The method according to any one of claims 1-9, further comprising an operation of determining the protein concentration of the recombinant batroxobin protein obtained via purification.
